# EUROPEAN PATENT APPLICATION

(11) **EP 4 524 982 A1**
(43) Date of publication of application: **19.03.2025**
(21) Application number: 23846051.3
(22) Date of filing: 14.06.2023
(51) Int. Cl.: G16H 20/00

(54) **COMPUTER PROGRAM, INFORMATION PROCESSING DEVICE, INFORMATION PROCESSING METHOD, AND LEARNING MODEL GENERATION METHOD**

(30) Priority: 25.07.2022 JP 2022118145
(71) Applicant: Terumo Kabushiki Kaisha, Tokyo 151-0072 (JP)
(72) Inventor: SHIMADA, Naoya, Ashigarakami-gun, Kanagawa 259-0151 (JP); KIKUCHI, Hideka, Ashigarakami-gun, Kanagawa 259-0151 (JP); YAGO, Seiji, Ashigarakami-gun, Kanagawa 259-0151 (JP); MARUYAMA, Tomoji, Ashigarakami-gun, Kanagawa 259-0151 (JP); TAKEUCHI, Takanori, Tokyo 163-1450 (JP); YOKOUCHI, Atsushi, Tokyo 163-1450 (JP); FUJII, Naoko, Ashigarakami-gun, Kanagawa 259-0151 (JP)
(74) Representative: KIPA AB
(86) International application number: PCT/JP2023/022149
(87) International publication number: WO 2024/024317

(57) **Abstract**

A computer program, an information processing device, an information processing method, and a learning model generation method that assist an appropriate pain treatment are provided.

The computer program causes a computer to execute processing including acquiring pain related information regarding pain of a patient, in a case where the pain related information is input, inputting the acquired pain related information into a learning model that outputs an index indicating an effect of a pain treatment and acquiring an index indicating an effect of a pain treatment regarding the patient, generating pain treatment assistance information for the patient based on the acquired index, and outputting the generated pain treatment assistance information.

## Description

### Technical Field

The present invention relates to a computer program, an information processing device, an information processing method, and a learning model generation method.

### Background Art

Patent Literature 1 discloses a medical information management system that includes a plurality of medical department servers that provides medical information stored in a database of a plurality of medical departments in a medical institution and a specific disease treatment integrated server that acquires specific disease medical information from the medical department server and provides the integrated specific disease medical information from an integrated database, in which a treatment progressing status can be grasped with high immediacy by making it possible to refer to medical information of specific diseases such as cancer in all treatment methods combining a surgery, radiation therapy, chemotherapy, and palliative care and registered related information of the specific diseases in a wide range.

### Citation List

### Patent Literature

Patent Literature 1: JP 2010-3135 A

### Summary of Invention

### Technical Problem

In a medical field, pain caused by diseases such as cancer is evaluated by pain measurement using an index such as a visual analog scale (VAS) or a device. These methods are cumbersome for a patient, and there is a problem that a doctor can only make evaluation only when the doctor is facing the patient. Furthermore, specialized knowledge is essential for effective pain management, and there is a problem that it is difficult for a doctor other than a specialist to perform an accurate pain treatment according to the patient.

The present invention has been made in view of the above circumstances, and an object of the present invention is to provide a computer program, an information processing device, an information processing method, and a learning model generation method that can assist an appropriate pain treatment.

### Solution to Problem

(1) A computer program according to the present invention causes a computer to execute processing including acquiring pain related information regarding pain of a patient, in a case where the pain related information is input, inputting the acquired pain related information into a learning model that outputs an index indicating an effect of a pain treatment and acquiring an index indicating an effect of a pain treatment regarding the patient, generating pain treatment assistance information for the patient based on the acquired index, and outputting the generated pain treatment assistance information.
   Here, in an embodiment of the present invention,
(2) in the computer program according to (1), the pain related information preferably includes at least one of an evaluation index used to evaluate pain, vital data, medication information, and a physical condition.
(3) The computer program according to (1) or (2), preferably causes the computer to execute processing further including acquiring medical information regarding medical care of the patient, and in a case where the medical information is further input, inputting the acquired medical information into the learning model that outputs the index indicating the effect of the pain treatment and acquiring the index indicating the effect of the pain treatment regarding the patient.
(4) The computer program according to any one of (1) to (3), in which the medical information preferably includes at least one of diagnosis information, examination information, treatment information, and prescription information.
(5) The computer program according to any one of (1) to (4), preferably causes the computer to execute processing further including acquiring literature information regarding a pain treatment, and in a case where the literature information regarding the pain treatment is further input, inputting the acquired literature information into the learning model that outputs the index indicating the effect of the pain treatment and acquiring the index indicating the effect of the pain treatment regarding the patient.
(6) The computer program according to any one of (1) to (5), in which the pain treatment assistance information preferably includes at least one of the index indicating the effect of the pain treatment used to assist a doctor who examines the patient, recommended prescription information, and recommended treatment information.
(7) The computer program according to any one of (1) to (6), preferably causes the computer to execute processing further including outputting the pain treatment assistance information to a doctor terminal device.
(8) The computer program according to any one of (1) to (7), preferably causes the computer to execute processing further including outputting a temporal change in the index indicating the effect of the pain treatment to the doctor terminal device.
(9) The computer program according to any one of (1) to (8), preferably causes the computer to execute processing further including outputting introduction information of a doctor having experience in a recommended treatment method to the doctor terminal device.
(10) An information processing device according to the present invention includes a first acquisition unit that acquires pain related information regarding pain of a patient, a second acquisition unit that inputs the acquired pain related information into a learning model that outputs an index indicating an effect of a pain treatment, in a case where the pain related information is input and acquires an index indicating an effect of a pain treatment regarding the patient, a generation unit that generates pain treatment assistance information for the patient based on the acquired index, and an output unit that outputs the generated pain treatment assistance information.
(11) An information processing method according to the present invention includes acquiring pain related information regarding pain of a patient, in a case where the pain related information is input, inputting the acquired pain related information into a learning model that outputs an index indicating an effect of a pain treatment and acquiring an index indicating an effect of a pain treatment regarding the patient, generating pain treatment assistance information for the patient based on the acquired index, and outputting the generated pain treatment assistance information.
(12) A learning model generation method according to the present invention includes acquiring first training data including pain related information regarding pain of a plurality of patients and an index indicating an effect of a pain treatment regarding the plurality of patients and generating a learning model so as to output the index indicating the effect of the pain treatment, in a case where the pain related information is input, based on the acquired first training data.
(13) The learning model generation method according to (12), in the learning model generation method according to supplementary note 12, preferably includes acquiring second training data further including medical information of the plurality of patients, literature information regarding the pain treatment, and the index indicating the effect of the pain treatment regarding the plurality of patients and generating the learning model so as to output the index indicating the effect of the pain treatment, in a case where the medical information and the literature information regarding the pain treatment are input, based on the acquired second training data.

### Advantageous Effects of Invention

According to the present invention, an appropriate pain treatment can be assisted.

### Brief Description of Drawings

Fig. 1 is a diagram illustrating an example of a configuration of a pain treatment assistance system according to the present embodiment.
Fig. 2 is a diagram illustrating an example of a configuration of a pain treatment assistance device.
Fig. 3 is a diagram illustrating information collection by the pain treatment assistance device.
Fig. 4A is a diagram illustrating an example of information in a database.
Fig. 4B is a diagram illustrating an example of the information in the database.
Fig. 4C is a diagram illustrating an example of the information in the database.
Fig. 5 is a diagram illustrating a first example of processing for generating pain treatment assistance information by the pain treatment assistance device.
Fig. 6 is a diagram illustrating a second example of the processing for generating the pain treatment assistance information by the pain treatment assistance device.
Fig. 7 is a diagram illustrating a third example of the processing for generating the pain treatment assistance information by the pain treatment assistance device.
Fig. 8 is a diagram illustrating an example of a learning model generation method.
Fig. 9 is a diagram illustrating a first example of the pain treatment assistance information.
Fig. 10 is a diagram illustrating a second example of the pain treatment assistance information.
Fig. 11 is a diagram illustrating a third example of the pain treatment assistance information.
Fig. 12 is a diagram illustrating a fourth example of the pain treatment assistance information.
Fig. 13 is a diagram illustrating a fifth example of the pain treatment assistance information.
Fig. 14 is a diagram illustrating a sixth example of the pain treatment assistance information.
Fig. 15 is a diagram illustrating an example of a processing procedure by the pain treatment assistance device.
Fig. 16 is a diagram illustrating an example of a learning model generation procedure.

### Description of Embodiments

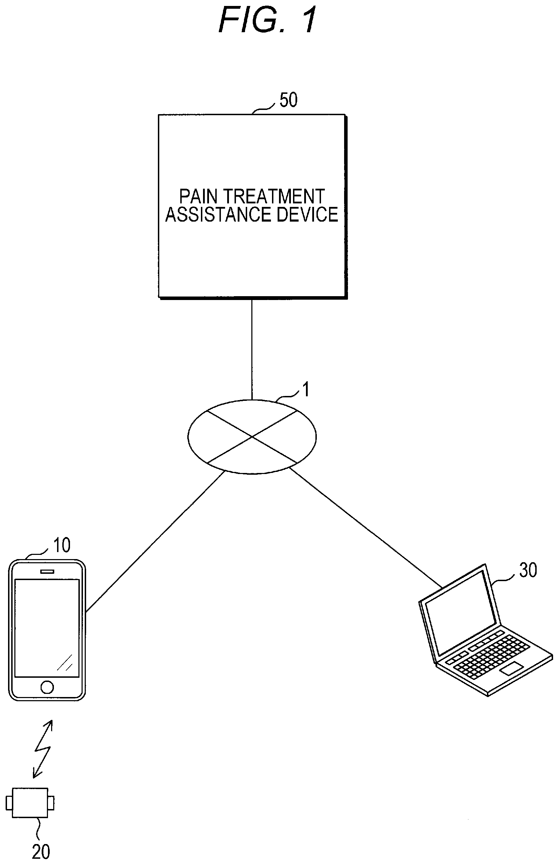

Hereinafter, an embodiment of the present invention will be described. Fig. 1 is a diagram illustrating an example of a configuration of a pain treatment assistance system according to the present embodiment. The pain treatment assistance system as an information processing system includes a pain treatment assistance device 50 as an information processing device. The pain treatment assistance system may include a patient terminal device 10, a device 20, and a doctor terminal device 30. The patient terminal device 10 and the doctor terminal device 30 are connected to the pain treatment assistance device 50 via a communication network 1. The device 20 may have a configuration that is wirelessly or wiredly connected to the patient terminal device 10 or may have a configuration that is connected to the pain treatment assistance device 50 via the communication network 1.

The patient terminal device 10 is a terminal device carried or held by a patient. The patient terminal device 10 can include a smartphone, a tablet, a personal computer, or the like including a display panel, an operation panel, a microphone, a speaker, or the like. In the patient terminal device 10, an app (application) for using the pain treatment assistance system is introduced. For example, a patient who is suffering from cancer or the like and periodically visits a medical institution while being treated at home is assumed. However, the patient is not limited to such a patient. Note that an assistant such as a family member or a caregiver may operate the patient terminal device 10, on behalf of the patient. Furthermore, the app may be a Web app or a downloaded app.

The device 20 is a measuring instrument that measures vital data of the patient and may be a wearable terminal or a stationary type. The device 20 can measure the vital data such as a body temperature, a heart rate, brain waves, or electrodermal activity (EDA). Note that the vital data is not limited to these, and may include any data assumed to be related to pain caused by the patient's disease. For example, a blood pressure, a pulse pressure, pulse waves, a blood sugar level, a weight, the number of steps, an activity amount, or the like may be included in the vital data.

The doctor terminal device 30 is a terminal device used by a doctor in a medical institution or the like. The doctor terminal device 30 can include a personal computer, a tablet, or the like including a display panel, an operation panel, or the like. A medical worker such as a public health nurse or a nurse may use the doctor terminal device 30 under guidance of the doctor. An application (for example, Web app or the like) for using the pain treatment assistance system is introduced in the doctor terminal device 30.

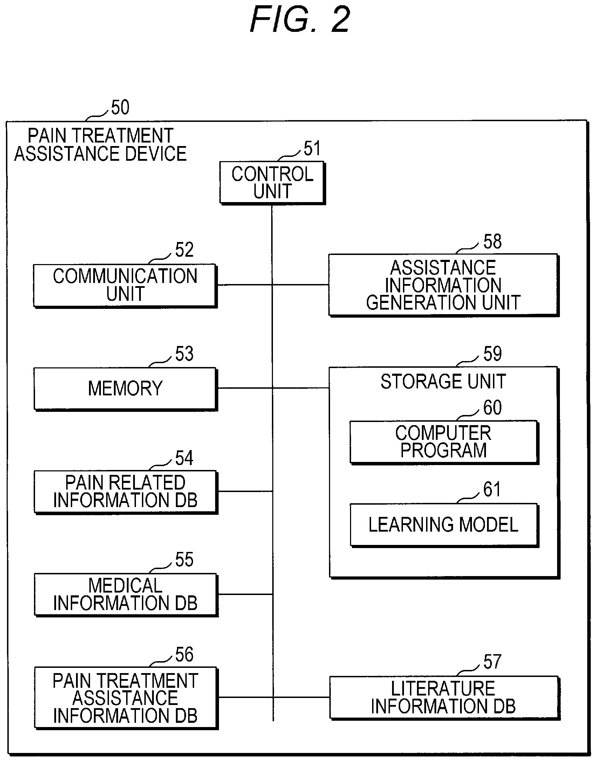

Fig. 2 is a diagram illustrating an example of a configuration of the pain treatment assistance device 50. The pain treatment assistance device 50 includes a control unit 51 that controls an entire device, a communication unit 52, a memory 53, a pain related information DB 54, a medical information DB 55, a pain treatment assistance information DB 56, a literature information DB 57, an assistance information generation unit (generation unit) 58, and a storage unit 59. The pain treatment assistance device 50 may distribute processing functions and include a plurality of devices. A database such as the pain related information DB 54, the medical information DB 55, the pain treatment assistance information DB 56, or the literature information DB 57 may be built in the pain treatment assistance device 50 or may be incorporated into an external data server. Furthermore, the storage unit 59 may be incorporated into an external data server.

The storage unit 59 can include, for example, a hard disk, a semiconductor memory, or the like, and stores a computer program 60 (program product), a learning model 61, and necessary information. The computer program 60 may be downloaded from an external device via the communication unit 52 and stored in the storage unit 59. Furthermore, the computer program 60 recorded in a recording medium (for example, optically readable disk storage medium such as CD-ROM) may be read by a recording medium reading unit and stored in the storage unit 59, or the computer program 60 may be read by the recording medium reading unit and developed in the memory 53.

The control unit 51 may be configured by incorporating a required number of central processing units (CPUs), micro-processing units (MPUs), graphics processing units (GPUs), or the like. The control unit 51 can execute processing defined by the computer program 60. That is, the processing executed by the control unit 51 corresponds to processing executed in accordance with the computer program 60. The control unit 51 can execute functions of the assistance information generation unit 58, by executing the computer program 60. The assistance information generation unit 58 may include hardware or software or may be implemented by a combination of hardware and software. The control unit 51 can execute processing using the learning model 61. Note that the control unit 51 has functions as a first acquisition unit, a second acquisition unit, a generation unit, and an output unit.

The communication unit 52 includes a communication module and can exchange necessary information with the patient terminal device 10 and the doctor terminal device 30 via the communication network 1. Furthermore, the communication unit 52 may exchange necessary information with the device 20.

The memory 53 may include a semiconductor memory, such as a static random access memory (SRAM), a dynamic random access memory (DRAM), or a flash memory. Loading the computer program 60 into the memory 53 enables the control unit 51 to execute the computer program 60.

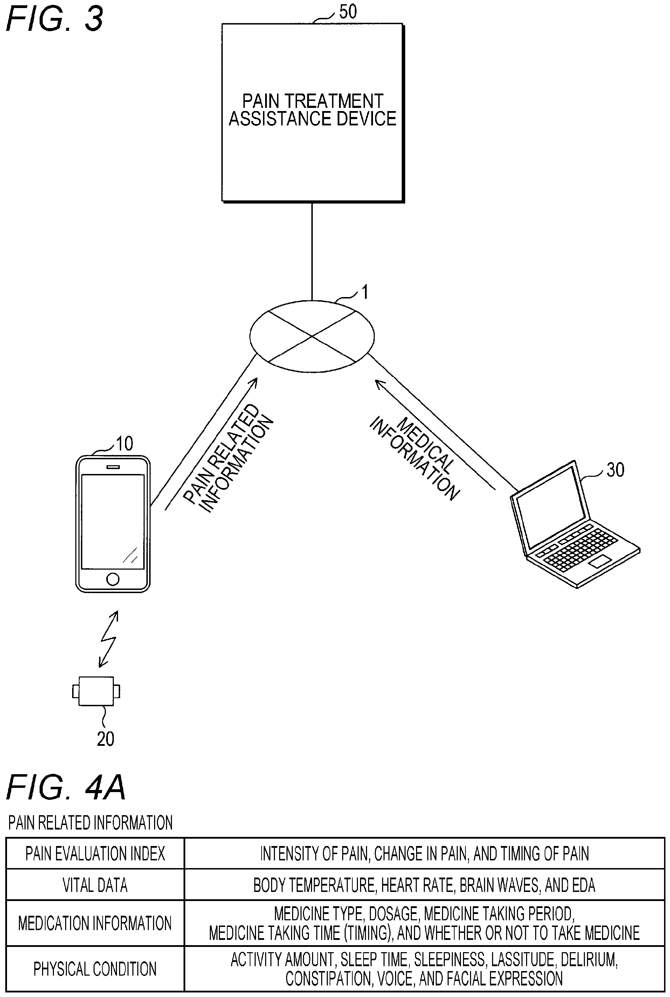

Fig. 3 is a diagram illustrating information collection by the pain treatment assistance device 50. The pain treatment assistance device 50 acquires pain related information related to pain caused by the patient's disease from the patient terminal device 10 and the device 20 via the communication unit 52. The pain related information is repeatedly transmitted from the patient terminal device 10 and the device 20 as appropriate. For example, the patient may transmit the pain related information once in a period from a previous hospital visit to a next hospital visit or may transmit the pain related information a plurality of times according to a length of the period. The acquired pain related information is classified for each patient and for each acquired time point and stored in the pain related information DB 54.

Furthermore, the pain treatment assistance device 50 acquires medical information related to medical care of the patient from the doctor terminal device 30 via the communication unit 52. The acquired medical information is classified for each patient and for each medical care time point and stored in the medical information DB 55.

Figs. 4A to 4C are diagrams illustrating an example of information in a database. Fig. 4A illustrates an example of information in the pain related information DB 54. The pain related information can be classified into a pain evaluation index (evaluation index used to evaluate pain), vital data, medication information, and a physical condition. The pain related information transmitted from the patient terminal device 10 and the device 20 includes at least one of the pain evaluation index, the vital data, the medication information, and the physical condition. Note that the classification is an example and is not limited to the example in Fig. 4A.

The pain evaluation index includes an intensity of pain, a change in the pain, a timing of the pain, or the like. The pain evaluation index can be transmitted from the patient terminal device 10 to the pain treatment assistance device 50, for example, when the patient activates the app of the patient terminal device 10 and answers a question regarding the intensity of the pain, the change in the pain, the timing of the pain, or the like.

The vital data includes the body temperature, the heart rate, the brain waves, the EDA, the pulse waves, or the like that changes in response to the pain. Note that the blood pressure, the pulse pressure, the blood sugar level, the weight, the number of steps, the activity amount, or the like may be included. The vital data can be acquired using the device 20.

The medication information includes a medicine type, a dosage, a medicine taking period, a medicine taking time (timing), whether or not to take the medicine, or the like. The medication information may include not only a medicine that is currently taken but also a medicine that has been taken in the past.

The physical condition includes an activity amount, a sleep time, sleepiness, lassitude, delirium, constipation, voice, facial expression, or the like. It is possible to include an item that can evaluate whether or not a disorder occurs due to the pain, the medicine, medical conditions, or the like.

The pain related information includes at least one of the pain evaluation index (evaluation index used to evaluate pain), the vital data, the medication information, and the physical condition.

By acquiring the pain related information as described above from the patient terminal device 10 and the device 20, the patient can accurately tell pain and symptoms that the patient feels at a necessary timing and a necessary frequency, in a period from a previous examination to a next examination.

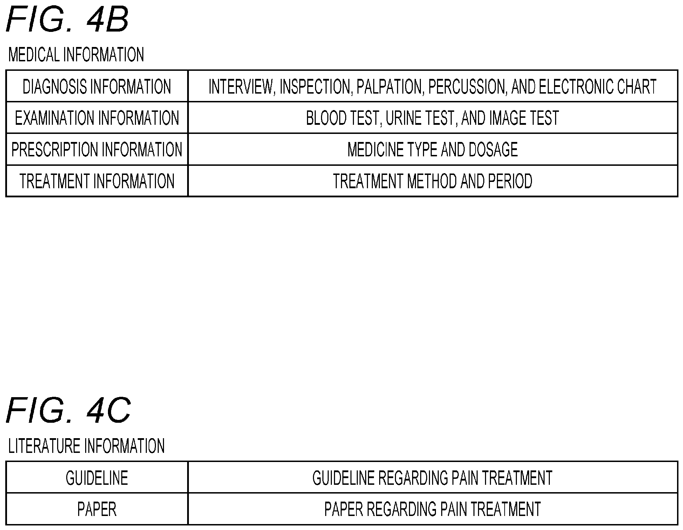

Fig. 4B illustrates an example of information in the medical information DB 55. The medical information can be classified into diagnosis information, examination information, prescription information, and treatment information. The medical information transmitted from the doctor terminal device 30 includes at least one of the diagnosis information, the examination information, the prescription information, and the treatment information. Note that the classification is an example and is not limited to the example in Fig. 4B.

The diagnosis information includes findings by interview, inspection, palpation, or percussion at the time of examination, an electronic chart, or the like. The examination information includes a blood test, a urine test, an image test, or the like. The prescription information includes a medicine type and a dosage prescribed by a doctor. Furthermore, the medicine taking period may be included. The treatment information includes a treatment method and a treatment period. Note that not only a literal treatment but also medicine prescription are included in a pain treatment.

Fig. 4C illustrates an example of information in the literature information DB 57. The literature information can be classified into a guideline and a paper. The guideline is a guideline regarding the pain treatment, and includes, for example, a guideline indicating what type of pain treatment is recommended for a symptom and pain of the patient. The paper is a paper regarding the pain treatment and includes, for example, objective information indicating what type of pain treatment is effective for the symptom and the pain of the patient. The literature information DB 57 is appropriately updated, and the latest information is recorded.

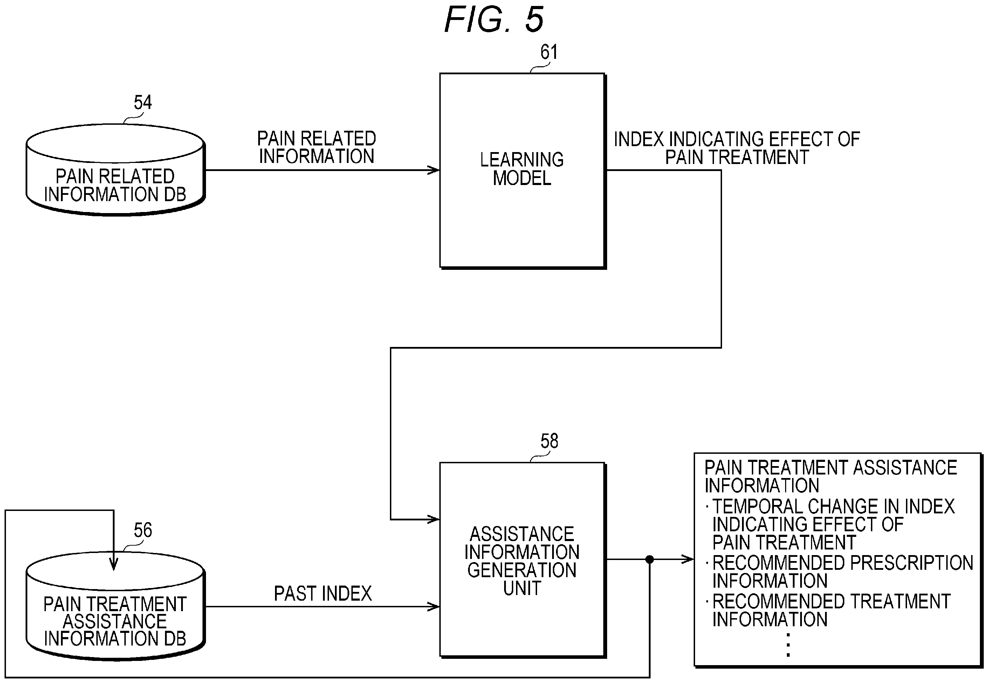

Fig. 5 is a diagram illustrating a first example of processing for generating pain treatment assistance information by the pain treatment assistance device 50. The control unit 51 reads pain related information of a specific patient from the pain related information DB 54 and inputs the read pain related information into the learning model 61. The pain related information of the specific patient is time-series data at a single or a plurality of time points. For example, not only current pain related information of the specific patient but also pain related information at a past time point going back from the current time point may be included. The control unit 51 can generate a pain related information vector by performing a vectorization operation for generating a primary vector for the pain related information of the patient and input the generated pain related information vector into the learning model 61. Note that the pain related information vector may be generated for each of the plurality of time points. Note that, although not illustrated, a past index of the pain treatment assistance information DB 56 may be input into the learning model 61.

The learning model 61 is generated (learned) to output an index indicating an effect of a current pain treatment, in a case where the pain related information is input. The index indicating the effect of the pain treatment is an index indicating a degree of the effect of the prescription or the treatment for the patient at the current time, and for example, may be expressed in several stages such as 10 stages from one to 10 or five stages from one to five. It is assumed that the pain of the patient be stronger as the index value is larger. Note that, it may be assumed that the pain of the patient be more alleviated, as the index value increases. Furthermore, the index may be classified and expressed such that the effect is appropriate, low, or high (side effects are concerned).

The learning model 61 can use, for example, a support vector machine (SVM), a decision tree, a random forest, an adaboost, a neural network (for example, recurrent neural network (RNN), long short term memory (LSTM), or the like), or the like.

The control unit 51 reads an index indicating an effect of a past pain treatment of the patient, recorded in the pain treatment assistance information DB 56. The control unit 51 inputs the read past index (index indicating effect of pain treatment) and the index acquired from the learning model 61 (index indicating effect of pain treatment) into the assistance information generation unit 58.

The assistance information generation unit 58 generates the pain treatment assistance information based on the input index (index indicating effect of pain treatment) and outputs the generated pain treatment assistance information. The assistance information generation unit 58 stores the generated pain treatment assistance information in the pain treatment assistance information DB 56. The pain treatment assistance information includes, for example, a temporal change in the index indicating the effect of the pain treatment of the patient, recommended prescription information or recommended treatment information for the patient, or the like. It is sufficient that the pain treatment assistance information include at least one of the index indicating the effect of the pain treatment used to assist the doctor who examines the patient, the recommended prescription information, and the recommended treatment information.

The temporal change in the index indicating the effect of the pain treatment includes, for example, information indicating a transition of the index of the patient from the past to the future. For example, it is sufficient that information indicate a change in the index values at a plurality of time points from a time of an initial diagnosis to the present. The recommended prescription information is information for assisting the doctor who examines the patient and includes information used to determine recommended prescription among medicine prescription for the patient. The recommended treatment information is information for assisting the doctor and includes information used to determine a recommended treatment method among treatment methods other than the medicine prescription for the patient.

The assistance information generation unit 58 may generate the pain treatment assistance information on a rule basis, for example, using a table indicating a correspondence relationship between the index indicating the effect of the pain treatment (time-series information) and the pain treatment assistance information. The table may indicate the correspondence relationship between the index indicating the effect of the pain treatment (time-series information) and the pain treatment assistance information.

Furthermore, the assistance information generation unit 58 can be configured by a model using a machine-learned algorithm and can use, for example, a support vector machine (SVM), a decision tree, a random forest, an adaboost, a neural network, or the like.

As described above, the control unit 51 can acquire the pain related information regarding the pain of the patient, input the acquired pain related information into the learning model 61 that outputs the index indicating the effect of the pain treatment, in a case where the pain related information is input and acquire the index indicating the effect of the pain treatment for the patient, generate the pain treatment assistance information for the patient based on the acquired index, and output the generated pain treatment assistance information.

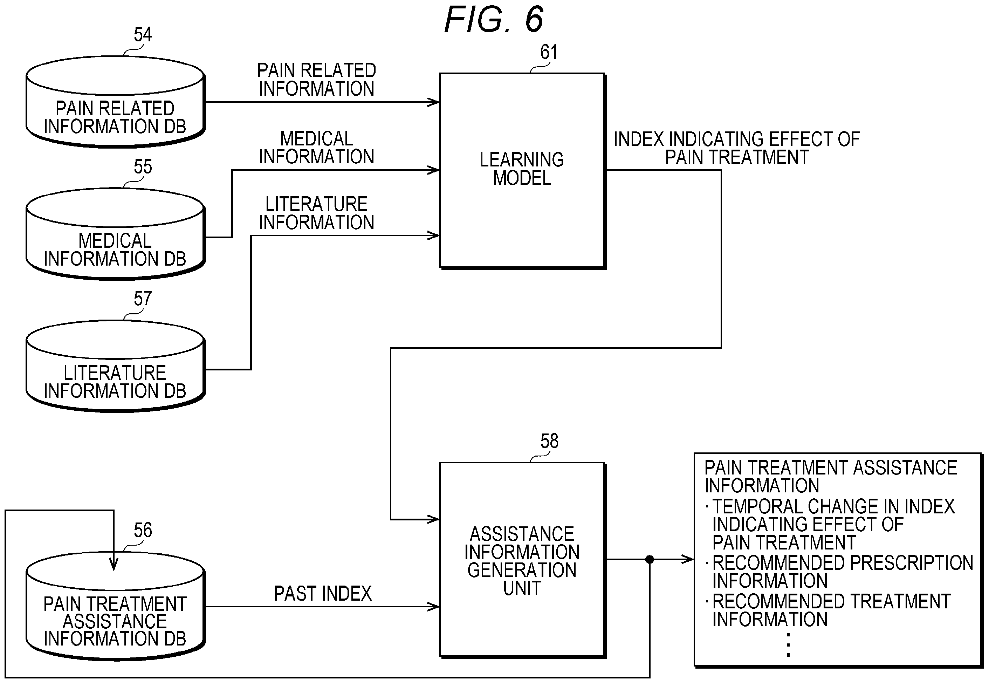

Fig. 6 is a diagram illustrating a second example of the processing for generating the pain treatment assistance information by the pain treatment assistance device 50. A difference from the first example illustrated in Fig. 5 is a point that the medical information and the literature information, in addition to the pain related information, are input into the learning model 61. The control unit 51 reads medical information of a specific patient from the medical information DB 55 and inputs the read medical information into the learning model 61. The medical information of the specific patient is time-series data at a single or a plurality of time points. For example, not only current medical information of the specific patient but also medical information at a past time point going back from the current time point may be included. The control unit 51 can perform the vectorization operation for generating the primary vector for the medical information, generate a medical information vector, and input the generated medical information vector into the learning model 61. Note that the medical information vector may be generated for each of the plurality of time points. Note that, although not illustrated, a past index of the pain treatment assistance information DB 56 may be input into the learning model 61.

Furthermore, the control unit 51 reads the literature information from the literature information DB 57 and inputs the read literature information into the learning model 61. The control unit 51 can perform the vectorization operation for generating the primary vector for the literature information, generate a literature information vector, and input the generated literature information vector into the learning model 61. It is expected that accuracy of an index output by the learning model 61 is improved by increasing the number of types of information to be input into the learning model 61.

As described above, the control unit 51 may acquire the medical information regarding the medical care of the patient, and input the acquired medical information into the learning model 61 that outputs the index indicating the effect of the pain treatment in a case where the medical information is further input and acquire the index indicating the effect of the pain treatment for the patient.

Furthermore, the control unit 51 may acquire the literature information regarding the pain treatment, and input the acquired literature information into the learning model 61 that outputs the index indicating the effect of the pain treatment in a case where the literature information regarding the pain treatment is further input and acquire the index indicating the effect of the pain treatment for the patient.

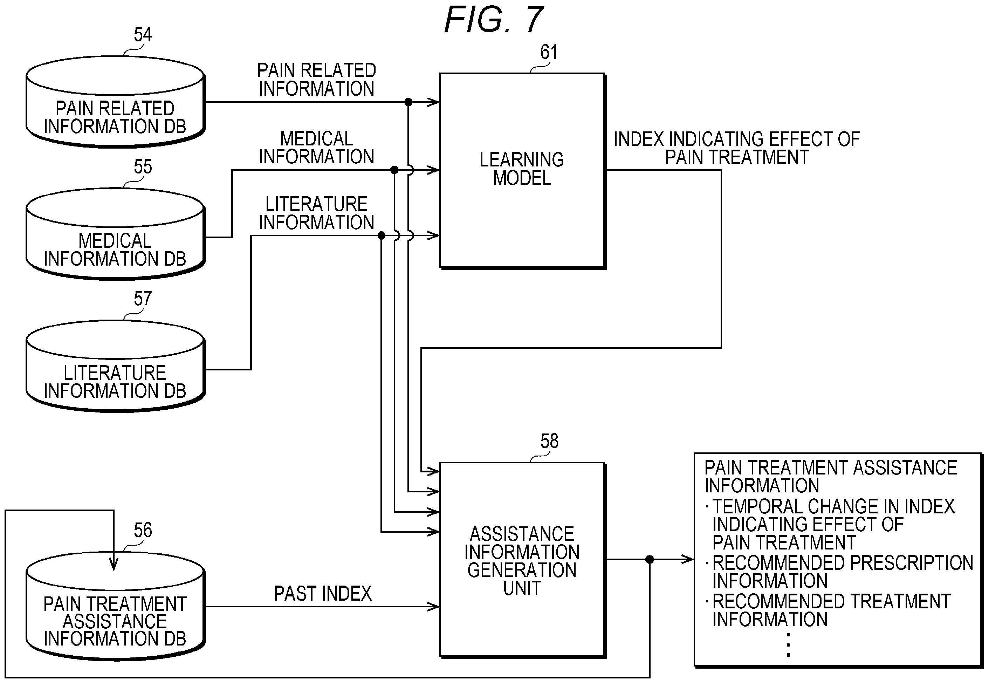

Fig. 7 is a diagram illustrating a third example of the processing for generating the pain treatment assistance information by the pain treatment assistance device 50. A difference from the second example illustrated in Fig. 6 is a point that the pain related information of the patient, the medical information, and the literature information are input into the assistance information generation unit 58, in addition to the index indicating the effect of the pain treatment. Note that, although not illustrated, a past index of the pain treatment assistance information DB 56 may be input into the learning model 61.

The assistance information generation unit 58 may generate the pain treatment assistance information on a rule basis, for example, using a table indicating a correspondence relationship between the index indicating the effect of the pain treatment (time-series information), the pain related information, the medical information and the literature information, and the pain treatment assistance information. The table may indicate the correspondence relationship between the index indicating the effect of the pain treatment (time-series information), the pain related information, the medical information, and the literature information, and the pain treatment assistance information.

Furthermore, as in the first and second examples, the assistance information generation unit 58 can be configured by the model using the machine-learned algorithm and can use, for example, a support vector machine (SVM), a decision tree, a random forest, an adaboost, a neural network, or the like.

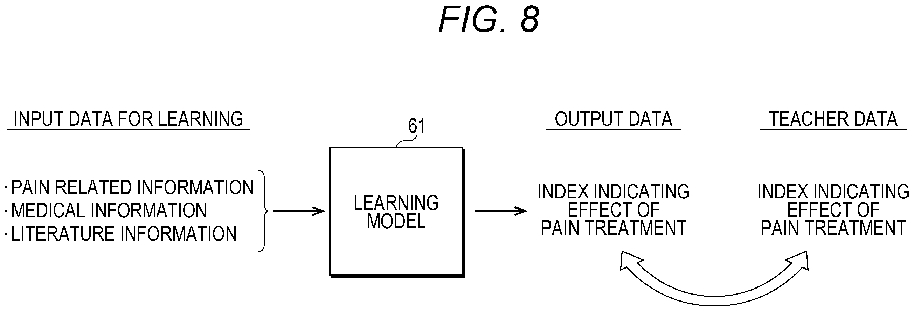

Fig. 8 is a diagram illustrating an example of a method for generating the learning model 61. Hereinafter, it is assumed that the pain treatment assistance device 50 generate the learning model 61. However, a learning processing device other than the pain treatment assistance device 50 may generate the learning model 61, and the pain treatment assistance device 50 may acquire the generated learning model 61. The control unit 51 acquires training data including the pain related information regarding the pain of the plurality of patients, the medical information, the literature information regarding the pain treatment, and the index indicating the effect of the pain treatment for the plurality of patients (second training data). The control unit 51 can generate the learning model 61 so as to output the index indicating the effect of the pain treatment, in a case where the pain related information, the medical information, and the literature information regarding the pain treatment are input, based on the acquired training data. At the time when the learning model 61 is generated, it is sufficient to adjust a parameter of the learning model 61 so that the index indicating the effect of the pain treatment, output from the learning model 61, approaches the index indicating the effect of the pain treatment that is teacher data included in the training data.

Note that, in the example in Fig. 8, the pain related information, the medical information, and the literature information are included in the input data for learning. However, for example, it is not necessary to include at least one of the medical information and the literature information in the input data for learning. For example, the control unit 51 can acquire the training data (first training data) including the pain related information regarding the pain of the plurality of patients and the index indicating the effect of the pain treatment for the plurality of patients and generate the learning model 61 so as to output the index indicating the effect of the pain treatment in a case where the pain related information is input, based on the acquired training data. However, it can be expected that inference accuracy of the learning model 61 is improved if at least one of the medical information and the literature information is included in the input data for learning.

Next, a specific example of the pain treatment assistance information will be described. In the following example, in a portion where input by the doctor is needed, voice input may be used. The control unit 51 outputs the pain treatment assistance information to the doctor terminal device 30. The pain treatment assistance information is displayed on the display panel of the doctor terminal device 30, and the doctor can perform an input operation or a selection operation according to displayed content.

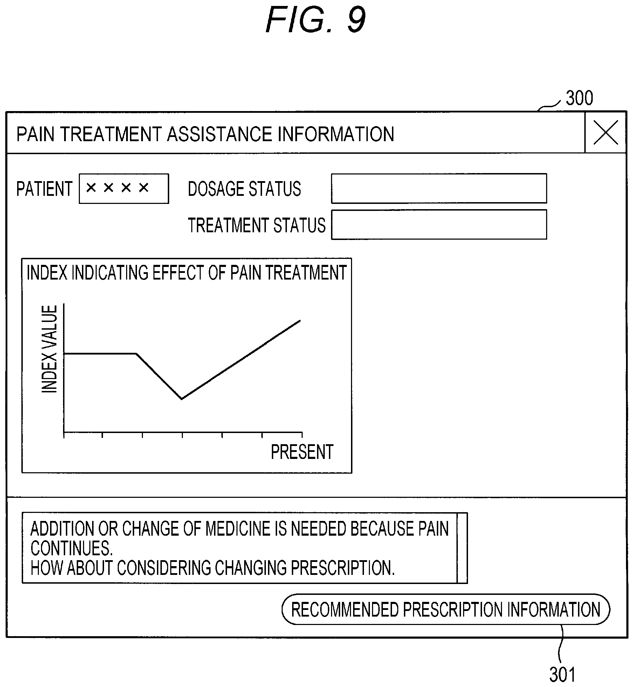

Fig. 9 is a diagram illustrating a first example of pain treatment assistance information 300. In the pain treatment assistance information 300, patient information (patient ID, patient name, or the like), a dosage status (medicine type, dosage, medicine taking period, or the like), a treatment status (treatment method, treatment period, or the like), and the index indicating the effect of the pain treatment are displayed. In the example in Fig. 9, the index indicating the effect of the pain treatment is displayed as a line graph, and a transition of the index value of the patient from the past to the present can be seen. In the example in Fig. 9, it is assumed that the pain of the patient be stronger as the index value is larger. The doctor can read that, in the pain treatment of the patient up to the present, although the pain is once alleviated, the pain is gradually getting stronger.

Furthermore, as an advice (assistance information) to the doctor, "Addition or change of medicine is needed because pain continues. How about considering changing prescription" is displayed. When the doctor operates a "recommended prescription information" icon 301, the recommended prescription information to be described later can be displayed.

As described above, the control unit 51 outputs a temporal change in the index indicating the effect of the pain treatment to the doctor terminal device 30.

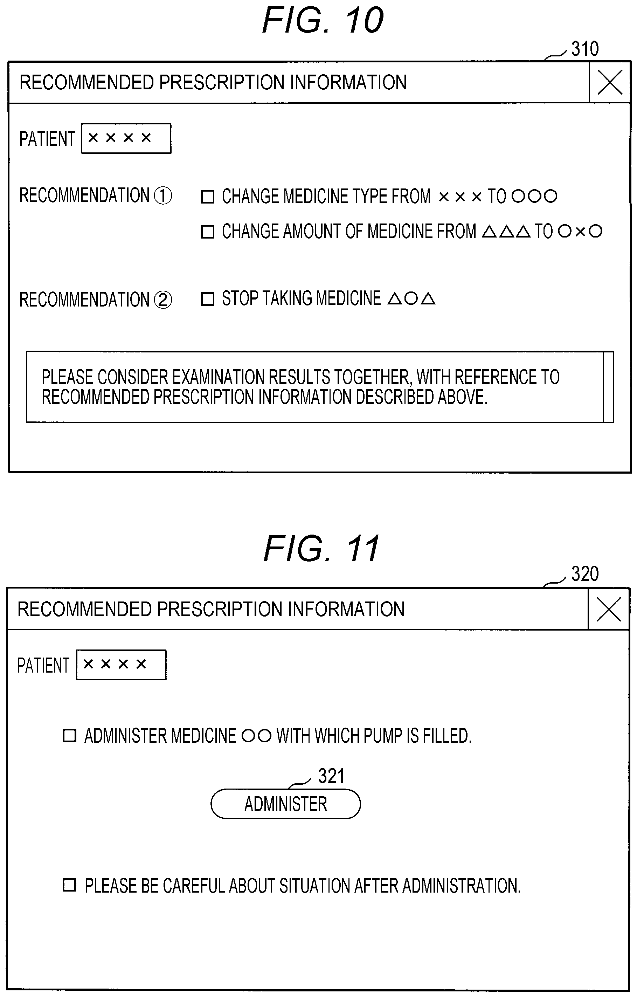

Fig. 10 is a diagram illustrating a second example of pain treatment assistance information 310. The pain treatment assistance information 310 includes the recommended prescription information. In the recommended prescription information, recommended content is displayed together with the patient information. In the example in Fig. 10, as a recommendation 1, a change in a medicine type and a change in a dosage are displayed. As a recommendation 2, it is recommended to stop taking a specific medicine. Furthermore, in addition to the recommended content, a guidance to the doctor may be displayed as "Please consider examination results together, with reference to recommended prescription information described above".

Fig. 11 is a diagram illustrating a third example of pain treatment assistance information 320. The pain treatment assistance information 310 includes the recommended prescription information. In the example in Fig. 11, "Administer medicine OO filled in pump" is displayed, and an "administer" icon 321 is displayed. The pump is, for example, a syringe pump or the like. Furthermore, in the recommended prescription information, a warning to the doctor is displayed as "Please be careful about situation after administration". When the doctor operates the "administer" icon 321, a medicine connected to the pump can be administered to the patient by the doctor's operation. Furthermore, an instruction may be transmitted to the medical worker, the caregiver, or the like so as to activate the pump at a remote place such as home. As a result, it is not necessary to remotely operate the pump.

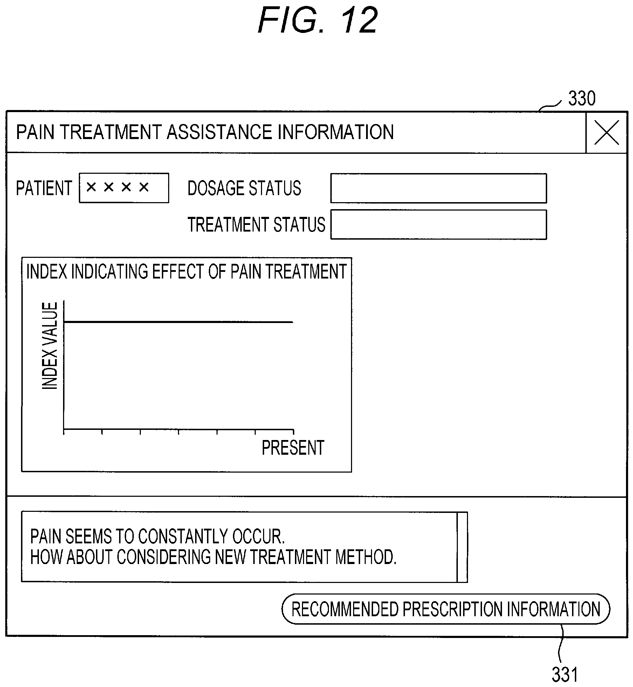

Fig. 12 is a diagram illustrating a fourth example of pain treatment assistance information 330. As compared with the first example in Fig. 9, in the fourth example, an index value transitions while remaining at a high value from the past to the present. In the example in Fig. 12, it is assumed that the pain of the patient be stronger as the index value is larger. The doctor can read that the pain treatment on the patient up to the present does not alleviate the pain.

Furthermore, as an advice (assistance information) to the doctor, "Pain seems to constantly occur. How about considering new treatment method" is displayed. When the doctor operates a "recommended prescription information" icon 331, the recommended prescription information to be described later can be displayed.

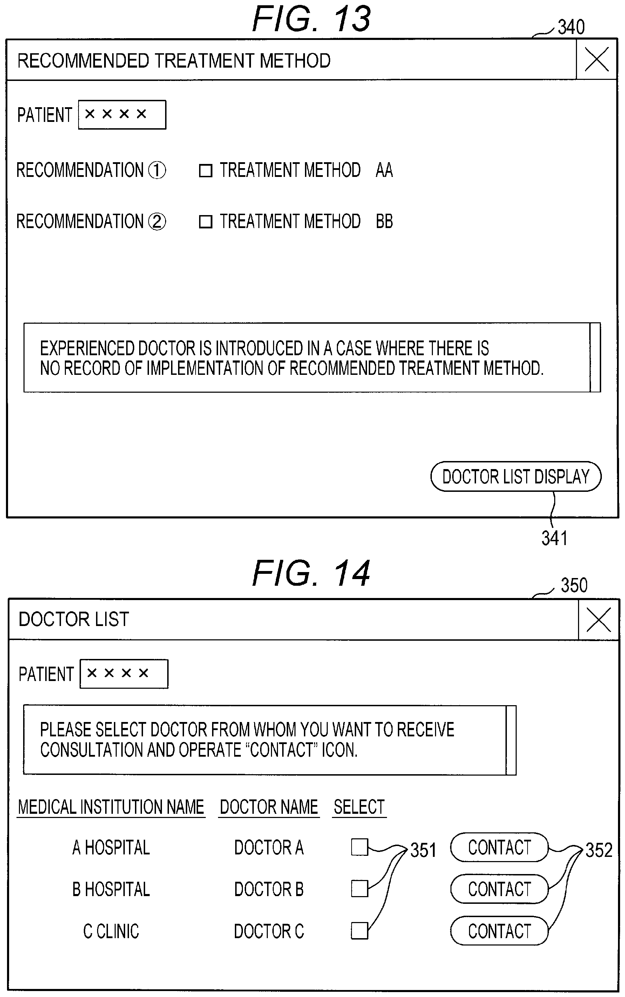

Fig. 13 is a diagram illustrating a fifth example of pain treatment assistance information 340. The pain treatment assistance information 340 includes a recommended treatment method. In the recommended treatment method, recommended content is displayed together with the patient information. In the example in Fig. 13, as a recommendation 1, a treatment method AA is displayed, and as a recommendation 2, a treatment method BB is displayed. Furthermore, in addition to the recommended content, "Experienced doctor is introduced in a case where there is no record of implementation of recommended treatment method" is displayed. When the doctor operates a "doctor list display" icon 341, introduction information of doctors having experience in the recommended treatment method is displayed in a list format.

Fig. 14 is a diagram illustrating a sixth example of pain treatment assistance information 350. In the pain treatment assistance information 350, a doctor list is displayed. The doctor list includes, for example, a medical institution name, a doctor name, a selection box 351, and a "contact" icon 352. As a guidance to the doctor, for example, "Please select doctor from whom you want to receive consultation and operate "contact" icon" is displayed. When the doctor select a desired medical institution and doctor name and operates the "contact" icon 352, the doctor can contact a specialist from whom the doctor desires to receive consultation.

As described above, the control unit 51 outputs the introduction information of the doctor having the experience in the recommended treatment method to the doctor terminal device 30.

As described above, according to the present embodiment, it is possible to support (assist) decision making on a pain treatment policy of the doctor, and even the doctor has little experience regarding the pain treatment, information necessary for the pain treatment is provided. Therefore, the doctor other than the specialist can perform an appropriate pain treatment.

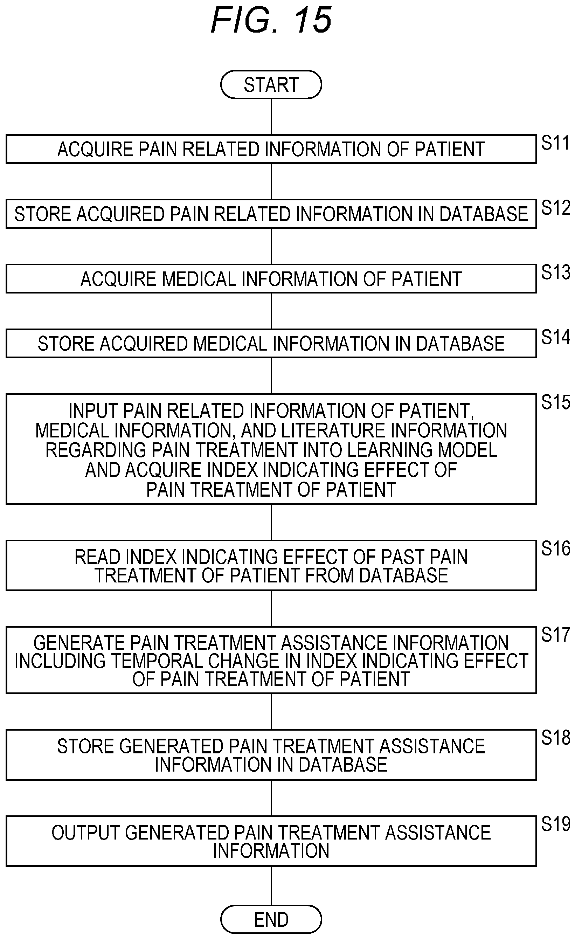

Fig. 15 is a diagram illustrating an example of a processing procedure by the pain treatment assistance device 50. Hereinafter, for convenience, the control unit 51 will be described as a subject of the processing. The control unit 51 acquires the pain related information of the patient (S11) and stores the acquired pain related information in the pain related information DB 54 (database) (S12). The pain related information can be repeatedly acquired at a required timing for each patient. For example, it can be acquired once or a plurality of times within a period from the time of the previous hospital visit to the time of the next hospital visit.

The control unit 51 acquires the medical information of the patient (S13) and stores the acquired medical information in the medical information DB 55 (database) (S14). The medical information can be acquired not only at the time of examination but also when the doctor determines that the medical information is needed.

The control unit 51 inputs the pain related information of the patient, the medical information, and the literature information regarding the pain treatment into the learning model 61 and acquires the index indicating the effect of the pain treatment of the patient, output from the learning model 61 (S15). The control unit 51 reads the index indicating the effect of the past pain treatment of the patient, from the pain treatment assistance information DB 56 (database) (S16).

The control unit 51 generates the pain treatment assistance information including the temporal change in the index indicating the effect of the pain treatment of the patient, based on the index indicating the effect of the pain treatment of the patient, read from the pain treatment assistance information DB 56 and the index indicating the effect of the pain treatment of the patient, acquired from the learning model 61 (S17). In this case, the pain treatment assistance information may be generated based on the pain related information of the patient, the medical information, and the literature information, in addition to the index indicating the effect of the pain treatment of the patient.

The control unit 51 stores the generated pain treatment assistance information in the pain treatment assistance information DB 56 (database) (S18), outputs the generated pain treatment assistance information (S19), and ends the processing.

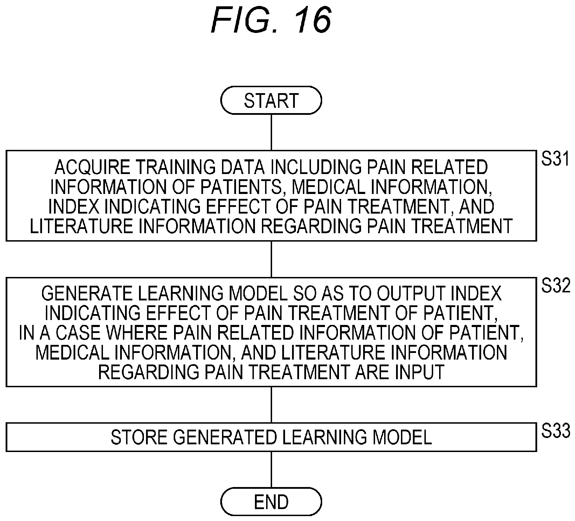

Fig. 16 is a diagram illustrating an example of a procedure for generating the learning model 61. The control unit 51 acquires training data including the pain related information regarding the pain of the plurality of patients, the medical information, the index indicating the effect of the pain treatment, and the literature information regarding the pain treatment (S31). The control unit 51 generates the learning model 61 so as to output the index indicating the effect of the pain treatment of each patient, in a case where the pain related information of each patient, the medical information, and the literature information regarding the pain treatment are input, based on the acquired training data (S32). The control unit 51 stores the generated learning model 61 in the storage unit 59 (S33) and ends the processing.

### Reference Signs List

- 1: communication network
- 10: patient terminal device
- 20: device
- 30: doctor terminal device
- 50: pain treatment assistance device
- 51: control unit
- 52: communication unit
- 53: memory
- 54: pain related information DB
- 55: medical information DB
- 56: pain management assistance information DB
- 57: literature information DB
- 58: assistance information generation unit
- 59: storage unit
- 60: computer program
- 61: learning model

## Claims

1. A computer program for causing a computer to execute processing comprising:
acquiring pain related information regarding pain of a patient;
in a case where the pain related information is input, inputting the acquired pain related information into a learning model that outputs an index indicating an effect of a pain treatment and acquiring an index indicating an effect of a pain treatment regarding the patient;
generating pain treatment assistance information for the patient based on the acquired index; and
outputting the generated pain treatment assistance information.

2. The computer program according to claim 1, wherein
the pain related information includes
at least one of an evaluation index used to evaluate pain, vital data, medication information, and a physical condition.

3. The computer program according to claim 1, for causing the computer to execute processing further comprising:
acquiring medical information regarding medical care of the patient; and
in a case where the medical information is further input, inputting the acquired medical information into the learning model that outputs the index indicating the effect of the pain treatment and acquiring the index indicating the effect of the pain treatment regarding the patient.

4. The computer program according to claim 3, wherein
the medical information includes
at least one of diagnosis information, examination information, treatment information, and prescription information.

5. The computer program according to claim 1, for causing the computer to execute processing further comprising:
acquiring literature information regarding a pain treatment; and
in a case where the literature information regarding the pain treatment is further input, inputting the acquired literature information into the learning model that outputs the index indicating the effect of the pain treatment and acquiring the index indicating the effect of the pain treatment regarding the patient.

6. The computer program according to any one of claims 1 to 5, wherein
the pain treatment assistance information includes
at least one of the index indicating the effect of the pain treatment used to assist a doctor who examines the patient, recommended prescription information, and recommended treatment information.

7. The computer program according to any one of claims 1 to 5, for causing the computer to execute processing further comprising:
outputting the pain treatment assistance information to a doctor terminal device.

8. The computer program according to any one of claims 1 to 5, for causing the computer to execute processing further comprising:
outputting a temporal change in the index indicating the effect of the pain treatment to a doctor terminal device.

9. The computer program according to any one of claims 1 to 5, for causing the computer to execute processing further comprising:
outputting introduction information of a doctor having experience in a recommended treatment method to a doctor terminal device.

10. An information processing device comprising:
a first acquisition unit that acquires pain related information regarding pain of a patient;
a second acquisition unit that inputs the acquired pain related information into a learning model that outputs an index indicating an effect of a pain treatment, in a case where the pain related information is input and acquires an index indicating an effect of a pain treatment regarding the patient;
a generation unit that generates pain treatment assistance information for the patient based on the acquired index; and
an output unit that outputs the generated pain treatment assistance information.

11. An information processing method comprising:
acquiring pain related information regarding pain of a patient;
in a case where the pain related information is input, inputting the acquired pain related information into a learning model that outputs an index indicating an effect of a pain treatment and acquiring an index indicating an effect of a pain treatment regarding the patient;
generating pain treatment assistance information for the patient based on the acquired index; and
outputting the generated pain treatment assistance information.

12. A learning model generation method comprising:
acquiring first training data including pain related information regarding pain of a plurality of patients and an index indicating an effect of a pain treatment regarding the plurality of patients; and
generating a learning model so as to output the index indicating the effect of the pain treatment, in a case where the pain related information is input, based on the acquired first training data.

13. The learning model generation method according to claim 12, further comprising:
acquiring second training data further including medical information of the plurality of patients, literature information regarding the pain treatment, and the index indicating the effect of the pain treatment regarding the plurality of patients; and
generating the learning model so as to output the index indicating the effect of the pain treatment, in a case where the medical information and the literature information regarding the pain treatment are input, based on the acquired second training data.
